Europäisches Patentamt

European Patent Office

Office européen des brevets

(19)

(11) Publication number: **0 132 450**
**B1**

(12) # EUROPEAN PATENT SPECIFICATION

(45) Date of publication of patent specification: **26.08.87**

(21) Application number: **83107292.1**

(22) Date of filing: **25.07.83**

(51) Int. Cl.⁴: **C 07 C 57/04,** C 07 C 51/487,
C 07 C 51/42, C 07 C 51/48

(54) **Method of purifying methacrylic acid.**

(43) Date of publication of application:
**13.02.85 Bulletin 85/07**

(45) Publication of the grant of the patent:
**26.08.87 Bulletin 87/35**

(84) Designated Contracting States:
**DE FR GB**

(56) References cited:
**US-A-2 288 281**
**US-A-2 394 572**
**US-A-2 457 257**

**S. PATAI: "The chemistry of the carbonyl
group", 1966, pages 388-390, chapter 8
"Chemical and physical methods of analysis",
Interscience Publishers, London, GB**

(73) Proprietor: **Nippon Shokubai Kagaku Kogyo
Co., Ltd
1, 5-chome, Koraibashi Higashi-ku
Osaka-shi Osaka-fu 541 (JP)**

(72) Inventor: **Shimizu, Noboru
11-20, Sakae-machi 2-chome
Takatsuki-shi Osaka-fu (JP)**
Inventor: **Daigo, Hiromiki
13-401, No. 2639, Aomadani
Minoo-shi Osaka-fu (JP)**
Inventor: **Yoshida, Hiroshi
21-7, Uenosaka 1-chome
Toyonaka-shi Osaka-fu (JP)**
Inventor: **Matsumoto, Shoichi
3-3, Ayaba, 2-chome
Ikeda-shi Osaka-fu (JP)**

(74) Representative: **Kraus, Walter, Dr. et al
Patentanwälte Kraus, Weisert & Partner
Thomas-Wimmer-Ring 15
D-8000 München 22 (DE)**

Courier Press, Leamington Spa, England.

## Description

This invention relates to a method for purifying methacrylic acid. More specifically, this invention relates to a method for obtaining purified methacrylic acid industrially efficiently without any trouble from an aqueous solution of methacrylic acid which is formed by cooling and condensing a methacrylic acid-containing reaction product gas from an oxidation reactor in a process for producing methacrylic acid by catalytically oxidizing isobutylene, tertiary butanol, methacrolein or isobutyraldehyde in the vapor phase.

It has been known from many literature references relating to the development of oxidation catalysts or oxidation processes that methacrylic acid can be produced by catalytically oxidizing a compound having 4 carbon atoms such as isobutylene or tertiary butanol in the vapor phase. In fact, however, methacrylic acid has not yet been produced industrially by a catalytic vapor-phase oxidation reaction. This is because no oxidation catalyst which has sufficient performance has been discovered, and the reaction product gas contains large amounts of by-products owing to the low performances of conventional catalysts. These by-products are extremely difficult to separate and remove from the desired methacrylic acid, and various troubles occur in the step of purifying methacrylic acid. The reaction product gas contains, in addition to methacrylic acid, various by-products such as methacrolein, acrolein, acrylic acid, acetic acid, acetaldehyde, carbon monoxide, carbon dioxide, maleic acid, aromatic carboxylic acids (e.g., benzoic acid and terephthalic acid), and tarry substances. In particular, the relatively high-boiling substances such as aromatic carboxylic acids and tarry substances are considered to cause various troubles in the isolating and purifying steps.

When methacrylic acid is to be isolated in the form of an aqueous solution by cooling and condensing the reaction product gas, the aforesaid many by-products dissolve in the aqueous solution of methacrylic acid. The relatively low boiling substances which are mainly composed of aldehydes such as methacrolein, acrolein and acetaldehyde in the aqueous solution of methacrylic acid are removed almost completely in a distilling or stripping step performed prior to the extracting step. But the relatively high-boiling substances such as maleic acid, aromatic carboxylic acids and tarry substances remain in the aqueous solution of methacrylic acid even after the distilling or stripping step, and may cause various troubles in the step of extracting methacrylic acid and a distilling step of separating methacrylic acid in pure form from the extract. To reduce these troubles, various proposals have been made in the past.

For example, Japanese Laid-Open Patent Publication No. 52021/1975 proposes a method which comprises treating the aqueous solution of methacrylic acid with activated carbon or a special ion exchanged resin, and Japanese Laid-Open Patent Publication No. 16438/1981 (European Patent Publication No. 23774A) proposes a method which comprises pre-mixing a solvent with the aqueous solution of methacrylic acid as a pre-treatment in order to separate the scum which deposits on the interface between the solvent and the aqueous layer at the time of extraction. These methods, however, only complicate the overall process, and do not give a complete solution to the problem. They are not industrially acceptable because the prevention of occurrence of the scum or its removal cannot be fully achieved and in a long-term operation, various troubles still occur, or large quantities of waste liquors are formed as a result of washing the apparatus.

US—A—2 394 572 describes a process for the purification of methacrylic acid in which the impure methacrylic solution is collected and then treated with an aqueous solution of an alkali metal bisulfide in order to remove the coloured impurities.

It is an object of this invention to provide a method for purifying methacrylic acid which can overcome the aforesaid defects.

According to this invention, there is provided, in a process for producing methacrylic acid by the catalytic vapor-phase oxidation of isobutylene, tertiary butanol, methacrolein or isobutyraldehyde, a method for purifying methacrylic acid which comprises subjecting an aqueous solution of methacrylic acid, obtained by cooling and condensing a methacrylic acid-containing reaction product gas which has left an oxidation reactor to a distilling or stripping step to remove low-boiling substances contained in the aqueous solution, adding a bisulfite salt, preferably an alkali metal bisulfite or ammonium bisulfite, to the resulting aqueous solution of methacrylic acid, and subjecting the mixture to an extracting step.

When isobutylene, tertiary butanol, methacrolein or isobutyraldehyde is oxidized in one or more stages by using an oxidation catalyst prepared from molybdenum oxide or a complex molybdenum oxide, a reaction product consisting mainly of methacrylic acid is obtained. Methacrylic acid is usually isolated as a condensed aqueous solution containing methacrylic acid. The aqueous solution of methacrylic acid is then subjected to a distillating or stripping operation in order to remove low-boiling substances and then subjected to an extraction operation with a solvent such as benzene, toluene, xylenes, acetic acid esters.

The present inventors extensively studied a method for separating and removing solid impurities (scums) deposited on the interface during the extraction of the aqueous solution of methacrylic acid from which the aforesaid low-boiling substances have been removed, and found that by adding a bisulfite salt to the aqueous solution of methacrylic acid before the extraction step, the occurrence of the scum is effectively inhibited, and in some cases, no scum is formed.

By performing the method of this invention, deposition of the scum on the upper interface in an extraction column can be prevented, and the adhesion of solid materials to the inner wall of the column, etc. can be prevented. Accordingly, the production of methacrylic acid can be operated for a long period of time without using a complicated procedure of removing the scum which is troublesome in the prior art. The present inventors have found that the scum deposited on the interface in the extraction column is composed of aromatic carboxylic acids, tarry substances and some amounts of polymers. But they have not determined why the addition of the bisulfite salt to the aqueous solution of methacrylic acid before the extraction step can lead to inhibition or prevention of scum deposition in the extracting step.

It has also been found surprisingly that by employing the method of this invention, the occurrence of methacrylic acid polymer and scales in the purifying step of obtaining pure methacrylic acid from the extract subsequent to the extracting step is drastically reduced. For example, the formation of a polymer in a distillation column for stripping the solvent from the extract or a rectifying column for purifying methacrylic acid can be inhibited, and the occurrence of scales on the heat-exchanger surface of a reboiler in the rectifying column for methacrylic acid owing to high-boiling substances is reduced. Furthermore, the amount of maleic acid in the extract is drastically decreased. The method of this invention, therefore, enables methacrylic acid of high purity to be obtained by a simple process.

Examples of the bisulfite salt used in this invention include bisulfite salts of alkali metals such as sodium, potassium and cesium and ammonium bisulfite. Sodium, potassium and ammonium bisulfites are preferred. The bisulfite is used in the form of an aqueous solution in a concentration of from 10% by weight to saturation. The amount of the bisulfite used is 0.5 to 15% by weight, preferably 1 to 10% by weight, based on the weight of methacrylic acid. When the amount of the bisulfite is too large, even methacrylic acid is converted to a salt and lost in the extracting step. Too small an amount is neither desirable because the effect of adding it is reduced. Preferably, the bisulfite salt is added by continuously mixing the aqueous solution of methacrylic acid and an aqueous solution of the bisulfite in a line-mixer, or mixing them continuously or semi-continuously in a stirred vessel, and then feeding the mixture to the extraction column. The temperature at the time of adding the bisulfite needs not to be particularly high, and room temperature suffices. If, desired, however, the adding operation may be carried out at a temperature of 50 to 60°C.

The aqueous solution of methacrylic acid to which the bisulfite salt has been added is then extracted in an extraction column with an ordinary solvent, for example aromatic hydrocarbons such as benzene, toluene and xylenes or esters such as ethyl acetate, propyl acetate, isopropyl acetate and butyl acetate. The extract obtained from the top of the column is directly fed to a solvent stripping column where the solvent is removed to give crude methacrylic acid. In the extracting operation, the extract may be washed with water in a customary manner.

In the step of distilling or stripping the relatively low-boiling substances, the step of stripping the solvent and the subsequent purifying step, the operation is preferably carried out in the presence of an ordinary polymerization inhibitor and molecular oxygen at a temperature of not more than 120°C under reduced pressure in order to prevent polymerization.

The following non-limitative Examples illustrate the present invention more specifically.

### Example 1

Tertiary butanol was oxidized with air in the vapor phase in the presence of steam using a first-stage reaction catalyst composed of a molybdenum-type complex oxide and a second-stage reaction catalyst based on a molybdenum-phosphorus type heteropolyacid. The resulting product gas was cooled and condensed to obtain an aqueous solution containing methacrylic acid. Low-boiling substances such as methacrolein were removed by distillation from the resulting aqueous solution to obtain an aqueous solution containing 24% by weight of acetic acid, 3.6% by weight of acetic acid, 1.4% by weight of phthalic acids (ortho-, meta- and para-), 0.8% by weight of maleic acid and 1.0% by weight of a tarry substance at a rate of 20 kg/hour.

A 30% by weight aqueous solution of sodium bisulfite was added to the aqueous solution at a rate of 0.5 kg/hour. The mixture was continuously fed into an extraction column from its top. In the meantime, xylene was continuously fed into the extraction tower from its bottom at a rate of 20 kg/hour. Thus, the mixture was extracted for 2 days. The extracting operation was carried out at room temperature and atmospheric pressure. The extraction column was a rotating disc column having an inside diameter of 70 mm and a total height of 1800 mm. Thus, the extract (organic layer) and the residue (aqueous layer) were obtained at a rate of 26 kg/hour and 14.5 kg/hour, respectively.

There was scarcely any deposition of a solid scum on the interface at the upper portion of the extraction column. During the subsequent distillation steps, i.e. the separation of the solvent from the extract, the separation of light ends and the separation of heavy ends, adhesion of high-boiling substances or polymer to the distillation columns or the heat exchanger surface of the reboilers was very little, and the process could be operated for a long period of time.

### Comparative Example 1

An extract (organic layer) and a raffinate (aqueous layer) were obtained at a rate of 25.5 kg/hour and 14.5 kg/hour, respectively, by operating in the same way as in Example 1 except that sodium bisulfite was not added.

Much scum was deposited on the interface at the upper portion of the extraction column. After continuous operation for 5 hours, a large amount of scum deposited on the upper viewing window portion, and the operation had to be stopped in 10 hours. Not much solid adhered to the columns or reboilers of the solvent separating column and the light ends separating column, but a very large amount of a solid adhered to the heat exchanger surface of the reboiler at the column for separating heavy ends.

### Example 2

An extract (organic layer) and a raffinate (aqueous layer) were obtained at a rate of 26 kg/hour and 15 kg/hour, respectively, by operating in the same way as in Example 1 except that a 30% by weight aqueous solution of ammonium bisulfate was added at a rate of 1.0 kg/hour instead of adding a 30% aqueous solution of sodium bisulfite at a rate of 0.5 kg/hour.

There was scarcely any deposition of a solid scum on the interface in the upper portion of the extracting column, and the adhesion of high-boiling substances or polymer to the columns or the heat exchanger surfaces of the reboilers in the distillation columns for separating the solvent from the extract and separating light ends and heavy ends respectively was very little. Accordingly, the process could be operated for a long period of time.

### Claims

1. In a process for producing methacrylic acid by catalytically oxidizing isobutylene, tertiary butanol, methacrolein or isobutyraldehyde in the vapor phase, a method for purifying methacrylic acid which comprises subjecting an aqueous solution of methacrylic acid, obtained by cooling and condensing a methacrylic acid-containing reaction product gas which has left an oxidation reactor, to a distilling or stripping step to remove low-boiling substances contained in the aqueous solution, adding a bisulfite salt to the resulting aqueous solution of methacrylic acid, and subjecting the mixture to an extracting step.

2. The method of claim 1 wherein the bisulfite salt is at least one member selected from the group consisting of bisulfite salts of alkali metals and ammonium bisulfite.

### Patentansprüche

1. Verfahren zur Reinigung von Methacrylsäure bei einem Verfahren zur Herstellung von Methacrylsäure durch katalytische Oxidation von Isobutylen, tert.-Butanol, Methacrolein oder Isobutyraldehyd in der Dampfphase, dadurch gekennzeichnet, daß man eine wäßrige Lösung von Methacrylsäure, erhalten durch Abkühlen und Kondensieren eines Methacrylsäure enthaltenden Reaktionsgases, das einen Oxidationsreaktor verlassen hat, einer Destillations-oder Abstreifstufe unterwirft, um niedersiedende Substanzen zu entfernen, die in der wäßrigen Lösung enthalten sind, daß man zu der resultierenden wäßrigen Lösung von Methacrylsäure ein Bisulfitsalz zusetzt und daß man das Gemisch einer Extraktionsstufe unterwirft.

2. Verfahren nach Anspruch 1, dadurch gekennzeichnet, daß das Bisulfitsalz mindestens eine Substanz, ausgewählt aus der Gruppe bestehend aus Bisulfitsalzen von Alkalimetallen und Ammoniumbisulfit, ist.

### Revendications

1. Dans un procédé de production d'acide méthacrylique par oxydation catalytique en phase vapeur de l'isobutylène, du butanol tertiaire, de la méthacroléine ou de l'isobutyraldéhyde, une méthode de purification de l'acide méthacrylique dans laquelle on soumet la solution aqueuse de l'acide qui a été obtenue par refroidissement et condensation du produit de réaction gazeux contenant l'acide méthacrylique, arrivant du réacteur d'oxydation, à une opération de distillation ou d'épuration pour en éliminer les substances à bas point di'ébullition, on ajoute un bisulfite minéral à la solution aqueuse d'acide méthacrylique et on soumet le mélange à une extraction.

2. La méthode de la revendication 1 dans laquelle le bisulfite est un bisulfite de métal alcalin ou d'ammomnium ou plusieurs de ces bisulfites.